# EUROPEAN PATENT APPLICATION

(11) **EP 4 442 837 A1**
(43) Date of publication of application: **09.10.2024**
(21) Application number: 22900920.4
(22) Date of filing: 12.10.2022
(51) Int. Cl.: C12Q 1/6844, G01N 33/543

(54) **ANALYSIS METHOD**

(30) Priority: 02.12.2021 JP 2021196186
(71) Applicant: DENSO CORPORATION, Kariya-shi, Aichi 448-8661 (JP)
(72) Inventor: NUKAZUKA, Akira, Kariya-shi, Aichi 448-8661 (JP); SAKAI, Teppei, Kariya-shi, Aichi 448-8661 (JP); ASANO, Mana, Kariya-shi, Aichi 448-8661 (JP); HAYAKAWA, Kei, Kariya-shi, Aichi 448-8661 (JP); NAKAGAWA, Kazuhisa, Kariya-shi, Aichi 448-8661 (JP); NIIMOTO, Mai, Kariya-shi, Aichi 448-8661 (JP)
(74) Representative: Kuhnen & Wacker Patent- und Rechtsanwaltsbüro PartG mbB
(86) International application number: PCT/JP2022/038105
(87) International publication number: WO 2023/100486

(57) **Abstract**

An analysis method according to the present disclosure includes: mixing a binding substance (1) and a sample (31) including a target substance (33). The binding substance includes a nucleic acid region (1) composed of a nucleic acid and having activity to bind to the target substance; removing the binding substance not bound to the target substance; amplifying the nucleic acid region; and detecting a phenomenon caused by the amplification of the nucleic acid region. For example, the analysis method includes: forming a complex (63) of a template nucleic acid (21, 21A, 21A-1) including a sequence complementary to a 3' side sequence of the nucleic acid region; and amplifying the nucleic acid region by an action of a nucleic acid amplifying enzyme using the complex as a starting point.

## Description

### CROSS REFERENCE TO RELATED APPLICATION

This application is based on Japanese Patent Application No. 2021-196186 filed on December 2, 2021, the disclosure of which is incorporated herein by reference.

### TECHNICAL FIELD

The present disclosure relates to an analysis method.

### BACKGROUND ART

Patent document 1 discloses an analysis method for a target substance by using a fusion body. The fusion body includes a binding substance and a labeled substance. The binding substance has activity to bind to the target substance. The labeled substance induces an observable phenomenon.

In the analysis method for the target substance, a sample containing the target substance is mixed with the fusion bodies. Fusion bodies, which have not been bound to the target substance, are then removed. Thereafter, the occurrence of the phenomenon caused by fusion bodies bound to the target substance is detected.

### PRIOR ART LITERATURES

### PATENT LITERATURE

Patent document 1 : JP 2019-33750 A

### SUMMARY

A fusion body is obtained by fusing a binding substance with a labeled substance. The fusion body has the following issues.

In order to produce a fusion body, it is necessary to perform steps of mixing the binding substance and the labeled substance, fusing them under appropriate conditions, and removing any remaining individual binding substance and labeled substance without the formation of any fusion bodies. Thus, the production of fusion bodies is costly and time-consuming. Further, there may occur variations in yield and purity between lots of the resulting fusion bodies.

Moreover, the formation of the fusion body may impair the original function of the binding substance or the original function of the labeled substance.

Furthermore, because the fusion body includes a labeled substance, the size of the fusion body is larger than the size of the binding substance. Thus, some fusion bodies may not be able to bind to the target substance due to interference from substances present in the vicinity of the target substance.

One aspect of the present disclosure provides an analysis method capable of detecting a target substance without using a fusion body including a labeled substance.

One of the present disclosure relates to an analysis method of a target substance. The analysis method includes: mixing a binding substance and a sample including a target substance. The binding substance includes a nucleic acid region composed of a nucleic acid and having activity to bind to the target substance. The analysis method includes: removing the binding substance not bound to the target substance; amplifying the nucleic acid region; and detecting a phenomenon caused by the amplification of the nucleic acid region.

According to the analysis method of the one aspect of the present disclosure, a target substance can be detected without using a fusion body including a labeled substance.

### BRIEF DESCRIPTION OF THE DRAWINGS

FIG. 1 is an explanatory diagram showing the structure of a nucleic acid aptamer and a single-stranded cyclic DNA.
FIG. 2 is an explanatory diagram showing the structure of a binding substance and the single-stranded cyclic DNA.
FIG. 3 is an explanatory diagram showing an analysis method for a target substance.
FIG. 4 is a graph showing changes over time in fluorescence intensity of mixed solutions X and Y.
FIG. 5 is a graph showing changes over time in fluorescence intensity of mixed solution Z.
FIG. 6 is a graph showing the amount of decrease in pH and the amount of DNA amplification expressed by the change in fluorescence intensity in a mixed solution Z.
FIG. 7 is a flowchart showing the steps of the analysis method for the target substance.

### DESCRIPTION OF EMBODIMENTS

Exemplary embodiments of the present disclosure will be described with reference to the drawings.

### 1. Binding substance 1

A binding substance 1 has activity to bind to a target substance 33. The target substance 33 is the substance to be analyzed. The target substance 33 is, for example, a protein, sugar, lipid, nucleic acid, or low molecular weight compound. The binding substance 1 includes a nucleic acid region 3 composed of a nucleic acid.

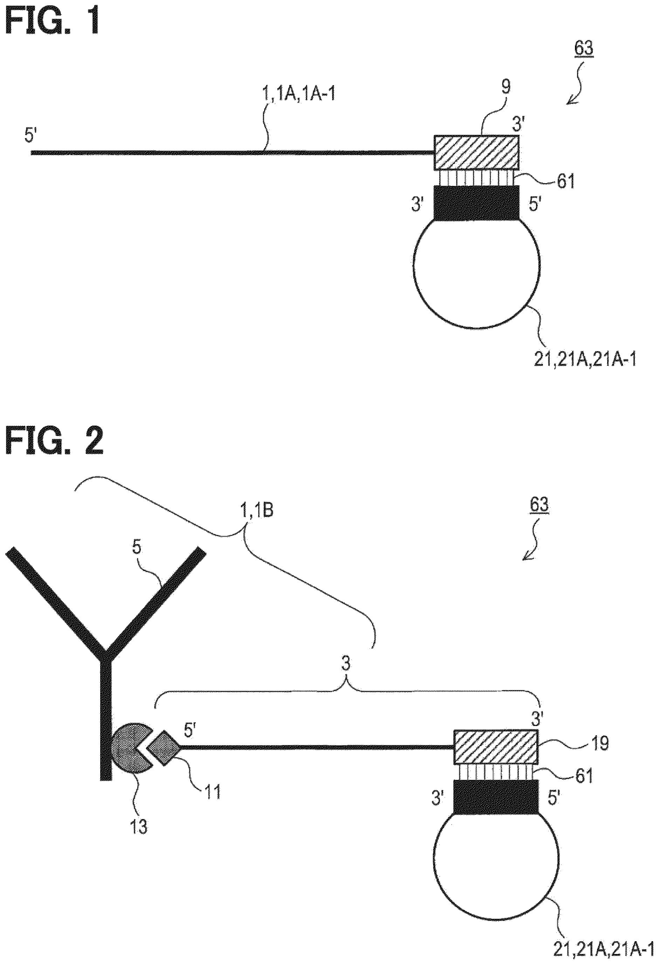

An example of the binding substance 1 is a nucleic acid preparation 1A. The nucleic acid preparation 1A includes, for example, nucleic acid aptamers 1A-1 shown in FIG. 1. The nucleic acid preparation 1A and the nucleic acid aptamers 1A-1 correspond to the nucleic acid region 3.

Examples of the nucleic acid aptamers 1A-1 include DNA aptamers and RNA aptamers. The nucleic acid aptamer 1A-1 can be chemically synthesized, for example, by an in-vitro process. The number of bases in the nucleic acid aptamer 1A-1 is, for example, 10 or more and 100 or less.

The nucleic acid aptamer 1A-1 may be composed of, for example, a plurality of linked units, each unit being composed of a nucleic acid. The number of bases of each unit is preferably 10 or more and 100 or less. When the number of bases of each unit is 100 or less, even if the target substance 33 is located close to other nucleic acid aptamers 1A-1 or other substances, the nucleic acid aptamer 1A-1 is less susceptible to interference from other nucleic acid aptamers 1A-1 or other substances and easily binds to the target substance 33.

The sequence of nucleic acid aptamer 1A-1 may be a DNA sequence, an RNA sequence, or a mixed sequence of DNA and RNA.

The nucleic acid aptamer 1A-1 may further contain modified nucleic acids or nucleic acid analogs as long as the activity to bind to the target substance 33, hybridization properties, and elongation properties are not impaired. A 3' terminal region 9 of the nucleic acid aptamer 1A-1 shown in FIG. 1 includes a base sequence complementary to a portion of a template nucleic acid 21. The 3' terminal region 9 is a region of the nucleic acid aptamer 1A-1 on the 3' end side. The 3' terminal region 9 preferably includes a base sequence in which the number of bases is 10 or more and 30 or less. The base sequence in the 3' terminal region 9 is not particularly limited. The GC content in the 3' terminal region 9 is preferably 30% or more and 70% or less.

The binding substance 1 is, for example, a binding substance 1B that artificially links a nucleic acid region 3 to a non-nucleic acid preparation 5 as illustrated in FIG. 2.

The non-nucleic acid preparation 5 is, for example, a protein preparation. Examples of the protein preparation include, for example, high molecular weight protein preparations and low molecular weight protein preparations. Examples of high molecular weight protein preparations include monoclonal antibodies, polyclonal antibodies, and the like. Examples of low molecule weight protein preparations include fragment antibodies, single chain antibodies, diabodies, nanobodies, VHHs, peptide aptamers, and the like.

The number of amino acids constituting the low molecular weight protein preparation is, for example, 5 or more and 200 or less. When the number of amino acids is 200 or less, even if the target substance 33 is located close to other low molecular weight protein preparations or other substances, the low molecular weight protein preparation is less susceptible to interference from other low molecular weight protein preparations or other substances and easily binds to the target substance 33.

The low molecular weight protein preparation may be composed of, for example, a plurality of linked units, each unit being composed of an amino acid. The number of amino acids in each unit is preferably 5 or more and 200 or less. When the number of amino acids in each unit is 200 or less, even if the target substance 33 is located close to other substances, the low molecular weight protein preparation is less susceptible to interference from other substances and easily binds to the target substance 33. The number of amino acids constituting the high molecular weight protein preparation is, for example, 200 or more.

The sequence of the nucleic acid region 3 may be a DNA sequence, an RNA sequence, or a mixed sequence of DNA and RNA. The nucleic acid region 3 may further contain modified nucleic acids or nucleic acid analogs as long as the activity of the binding substance 1B to the target substance 33, hybridization properties, and elongation properties are not impaired.

The number of bases and sequence in the nucleic acid region 3 are not particularly limited. The number of bases in the nucleic acid region 3 is preferably 15 or more and 40 or less. A 3' terminal region 19 of the nucleic acid region 3 shown in FIG. 2 includes a base sequence complementary to a portion of the template nucleic acid 21. The 3' terminal region 19 is a region of the nucleic acid region 3 on the 3' end side.

The 3' terminal region 19 preferably includes a base sequence having 10 to 30 bases. The base sequence of the 3' terminal region 19 is not particularly limited. The GC content in the 3' terminal region 19 is preferably 30% or more and 70% or less.

As shown in FIG. 2, the 5' end of the nucleic acid region 3 is chemically modified with a chemical substituent 11, for example, for artificial linkage with the non-nucleic acid preparation 5.

A method of artificially linking the non-nucleic acid preparation 5 and the nucleic acid region 3 is, for example, a method which involves chemically bonding a chemical substituent 13 of the non-nucleic acid preparation 5 to the chemical substituent 11 of the nucleic acid region 3.

Examples of the chemical substituent 11 or 13 include primary amine, azide, alkyne, dibenzocyclooctyne, bicyclononyne, 2-propynyl, 2'-O-propargyl, thiol, biotin, avidin, streptavidin, neutravidin, N-hydroxysuccinimide, maleimide, etc.

### 2. Template Nucleic Acid 21

The template nucleic acid 21 includes a base sequence complementary to the 3' terminal region 9 of the nucleic acid aptamer 1A-1 shown in FIG. 1 or the 3' terminal region 19 of the nucleic acid region 3 shown in FIG. 2.

For example, as shown in FIG. 1, the template nucleic acid 21 and the nucleic acid aptamer 1A-1 hybridize by forming a base pair 61 to form a complex 63. For example, as shown in FIG. 2, the template nucleic acid 21 and the nucleic acid region 3 hybridize by forming a base pair 61 to form a complex 63. The total number of bases in the template nucleic acid 21 is preferably 50 or more and 100 or less.

The template nucleic acid 21 is, for example, a single-stranded cyclic nucleic acid 21A. The single-stranded cyclic nucleic acid 21A is, for example, the single-stranded cyclic DNA 21A-1 shown in FIGS. 1 and 2. The single-stranded cyclic DNA 21A-1 is obtained by cyclizing the single-stranded linear DNA. Cyclization of single-stranded linear DNA can be performed using a DNA ligase such as CircLigase (Lucigen Corporation), CircLigase II (Lucigen Corporation), or T4 DNA Ligase (NEB Inc. and other companies).

### 3. Analysis method for the target substance 33

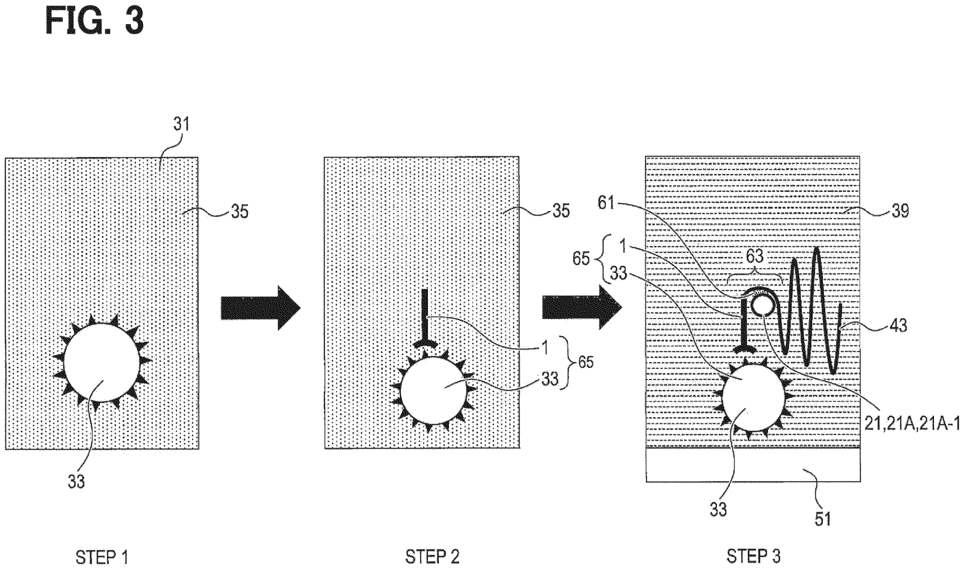

The analysis method for the target substance 33 can be performed, for example, by the following procedure. As shown in STEP 1 of FIG. 3, a sample 31 is prepared. The sample 31 contains the target substance 33 and a reaction solution 35 for conjugate formation. The target substance 33 is present in the reaction solution 35 for conjugate formation.

Next, as shown in STEP 2 of FIG. 3, the sample 31 and the binding substance 1 are mixed. The binding substance 1 is, for example, the nucleic acid aptamer 1A-1 shown in FIG. 1 or the binding substance 1B shown in FIG. 2. As a result, the target substance 33 and the binding substance 1 coexist in the reaction solution 35 for conjugate formation. At least a portion of the binding substance 1 and the target substance 33 are bound together to form a conjugate 65.

Next, a portion of the binding substance 1 that is not bound to the target substance 33 is removed. The reaction solution 35 for conjugate formation is also removed.

Next, as shown in STEP 3 of FIG. 3, a reaction solution 39 for nucleic acid amplification is added in place of the reaction solution 35 for conjugate formation. The reaction solution 39 for nucleic acid amplification contains known components of the strand displacement DNA synthetase, template nucleic acid 21, and reaction solution 39 for nucleic acid amplification. Examples of the strand displacement DNA synthetase include a phi29 polymerase, a Vent DNA polymerase, a Bst DNA polymerase, and the like. The strand displacement DNA synthetase corresponds to a nucleic acid amplifying enzyme.

The composition of the reaction solution 39 for nucleic acid amplification can be adjusted as appropriate according to the phenomenon to be detected and the like. The phenomenon is a phenomenon caused by amplifying the 3' terminal region 9 shown in FIG. 1 or the 3' terminal region 19 shown in FIG. 2. For example, the molar concentration or pH of a buffer solution in the reaction solution 39 for nucleic acid amplification can be adjusted. After adding the reaction solution 39 for nucleic acid amplification, the target substance 33 and the binding substance 1 bound to the target substance 33 are present in the reaction solution 39 for nucleic acid amplification.

By the action of the complementary sequence included in the template nucleic acid 21, the base pair 61 is formed between the binding substance 1 and the template nucleic acid 21, thereby forming the complex 63. Using the complex 63 as a starting point, the nucleic acid amplification occurs by the action of the nucleic acid amplifying enzyme. Nucleic acid amplification is the amplification of the 3' terminal region 9 shown in FIG. 1 or the 3' terminal region 19 shown in FIG. 2, the 3' terminal region 9 or 19 being included in the binding substance 1.

When the template nucleic acid 21 is single-stranded cyclic DNA 21A-1, the nucleic acid amplification using rolling circle amplification (RCA) is caused by the action of the strand displacement DNA synthetase.

The nucleic acid amplification using the rolling circle amplification can be performed under isothermal conditions. When phi29 polymerase is used as the strand displacement DNA synthetase, the reaction of the nucleic acid amplification preferably proceeds at a constant temperature of 30°C or higher and 40°C or lower. The nucleic acid amplification generates an amplified nucleic acid 43.

When amplifying the nucleic acid region by the rolling circle amplification, the reaction solution 39 for nucleic acid amplification preferably contains a tris buffer solution at a final concentration of 0 mM or more and 10 mM or less. When amplifying the nucleic acid region by the rolling circle amplification, the pH of the reaction solution 39 for nucleic acid amplification is preferably 7.0 or higher and 9.0 or lower. When the composition and pH of the reaction solution 39 for nucleic acid amplification are set as described above, changes in pH of the reaction solution 39 for nucleic acid amplification can be easily detected.

Isothermal nucleic acid amplification is preferred as the nucleic acid amplification. In addition to the rolling circle amplification method, examples of isothermal nucleic acid amplification include loop-mediated isothermal amplification (LAMP), whole genome amplification (WGA), multiple displacement amplification (MDA), nicking endonuclease amplification reaction (NEAR), and the like.

The isothermal nucleic acid amplification does not require a temperature cycle including temperature rise and fall, and its reaction proceeds at a constant temperature. Thus, the application of the isothermal nucleic acid amplification to a simple detection method becomes easy, compared to a polymerase chain reaction (PCR).

The reaction solution 39 for nucleic acid amplification contains, for example, a nucleic acid detection reagent. Examples of nucleic acid detection reagents include SYBR Green I, SYBR Green II, and the like. In a case where the reaction solution 39 for nucleic acid amplification contains the nucleic acid detection reagent, when nucleic acid amplification occurs, the reaction solution 39 for nucleic acid amplification is excited by light with a specific wavelength and emits fluorescence. The fluorescence corresponds to the phenomenon caused by the amplification of the nucleic acid region. The intensity of the fluorescence becomes higher as the amount of the binding substance 1 bound to the target substance 33 increases. The intensity of the fluorescence becomes higher as the amount of the target substance 33 contained in the sample 31 increases.

The intensity of fluorescence at the specific wavelength is increased in parallel with the nucleic acid amplification by using, for example, an intercalating luminescent dye or minor groove luminescent dye. The fluorescence at the specific wavelength corresponds to the phenomenon caused by the amplification of the nucleic acid region 3. The intensity of the fluorescence at the specific wavelength becomes higher as the amount of the binding substance 1 bound to the target substance 33 increases. The intensity of the fluorescence with the specified wavelength becomes higher as the amount of the target substance 33 contained in the sample 31 increases.

In the reaction solution 39 for nucleic acid amplification, nucleotides are incorporated into an amplified nucleic acid 43 along with the nucleic acid amplification, whereby hydrogen ions are generated. The amount of hydrogen ions generated increases in accordance with the amount of the nucleotides incorporated. The pH of the reaction solution 39 for nucleic acid amplification is decreased because of the generation of the hydrogen ions. The generation of the hydrogen ions and the decrease in pH correspond to the phenomena caused by the amplification of the nucleic acid region. The amount of decrease in pH becomes greater as the amount of the binding substance 1 bound to the target substance 33 increases. The amount of decrease in pH becomes greater as the amount of the target substance 33 contained in the sample 31 increases.

In the reaction solution 39 for nucleic acid amplification, nucleotides are incorporated into an amplified nucleic acid 43 along with the nucleic acid amplification, whereby pyrophosphoric acid is generated. When a group of enzymes catalyzing a reaction cascade driven by the pyrophosphoric acid is included in the reaction solution 39 for nucleic acid amplification, the reaction cascade driven by the generated pyrophosphoric acid causes luminescence and changes in oxidation-reduction potential. The generation of the pyrophosphoric acid, the luminescence, and the change in oxidation-reduction potential correspond to the phenomena caused by the amplification of the nucleic acid region. The extents of luminescence and change in the oxidation-reduction potential become greater as the amount of the binding substance 1 bound to the target substance 33 increases. The extents of luminescence and change in the oxidation-reduction potential become greater as the amount of the target substance 33 contained in the sample 31 increases.

In a case where a phenomenon caused by the nucleic acid amplification is a change in the oxidation-reduction potential, the phenomenon caused by the nucleic acid amplification can be detected using a potentiometer. In a case where the phenomenon caused by the nucleic acid amplification is the production, consumption, or absorption of hydrogen ions, the phenomenon caused by the nucleic acid amplification can be detected using a pH meter.

The phenomenon caused by the nucleic acid amplification can be detected by a measuring instrument 51 shown in STEP 3 of FIG. 3. The measuring instrument 51 is, for example, a light receiving device, a pH meter, a potentiometer, or the like.

### 4. Effects exhibited by analysis method

(1A) The binding substance 1 does not have to include a labeled substance. That is, according to the analysis method of the present disclosure, the target substance 33 can be detected without using a fusion body including a labeled substance. Thus, according to the analysis method of the present disclosure, the producing cost and time required for the production of the binding substance 1 can be reduced.

(1B) When the nucleic acid preparation 1A, such as a DNA aptamer or RNA aptamer, is used as the binding substance 1, the base sequence of the nucleic acid preparation 1A can be used as a template or primer part for the nucleic acid amplification without any modification in its original state. Thus, the nucleic acid preparation 1A can have both the function of serving as the binding substance 1 and the function of causing the phenomenon.

(1C) The binding substance 1 does not include a labeled substance. Thus, the original function of the binding substance 1 is difficult to eliminate. The original function of the binding substance 1 is the activity to bind to the target substance 33.

(1D) The binding substance 1 does not include a labeled substance. Thus, the size of the binding substance 1 is small. As a result, when the binding substance 1 approaches one target substance 33, it is less susceptible to interference from other binding substances 1. Therefore, the binding substance 1 can bind to, for example, each of a plurality of target substances 33 forming a complex. As a result, the analysis method of the present disclosure can be used to improve the analytical sensitivity of the target substances 33.

(1E) According to the analysis method of the present disclosure, the phenomenon associated with the nucleic acid amplification can be detected by amplifying the nucleic acid region following the formation of the conjugate 65 of the target substance 33 and the binding substance 1. Thus, the concentration of the target substance 33 in the sample 31 can be measured.

(1F) In the analysis method of the present disclosure, the concentration of hydrogen ions in the reaction solution 39 for nucleic acid amplification changes, for example, as a result of the nucleic acid amplification. In the analysis method of the present disclosure, for example, the change in the concentration of hydrogen ions caused in the reaction solution 39 for nucleic acid amplification can be measured using the pH meter. In such a case, the target substance 33 can be detected based on the measurement results of the pH meter.

(1G) In the analysis method of the present disclosure, the concentration of pyrophosphoric acid in the reaction solution 39 for nucleic acid amplification changes, for example, as a result of the nucleic acid amplification. The change in the oxidation-reduction potential is caused by the reaction cascade driven by the generated pyrophosphoric acid. In the analysis method of the present disclosure, for example, a change in the concentration of pyrophosphoric acid caused in the reaction solution 39 for nucleic acid amplification can be measured based on a change in the amount of luminescence and a change in the oxidation-reduction potential. In such cases, the target substance 33 can be detected based on changes in luminescence and oxidation-reduction potential.

(1H) In the analysis method of the present disclosure, the coloration, luminescence, or fluorescence caused in the reaction solution 39 for nucleic acid amplification can be measured, for example, by using the intercalating luminescent dye or minor groove luminescent dye that is adsorbed onto the amplified nucleic acid 43. In the analysis method of the present disclosure, for example, the target substance 33 can be detected based on the measurement results of the coloration, luminescence, or fluorescence caused in the reaction solution 39 for nucleic acid amplification.

(1I) In the analysis method of the present disclosure, for example, only the binding substance 1 bound to the target substance 33 is subject to the nucleic acid amplification. In such a case, as the concentration of the target substance 33 increases, the coloring intensity, luminescence intensity, fluorescence intensity, amount of change in the oxidation-reduction potential, or amount of change in the pH, which is caused in the reaction solution 39 for nucleic acid amplification, becomes more significant. Therefore, according to the analysis method of the present disclosure, the concentration of the target substance 33 can be quantitatively analyzed immunometrically.

(1J) In the analysis method of the present disclosure, for example, the nucleic acid amplification is performed at an isothermal temperature. In a case where the nucleic acid amplification is performed at the isothermal temperature, the reaction proceeds at a constant temperature. In a case where the nucleic acid amplification is performed at the isothermal temperature, the analysis method of the present disclosure is applied to a simple detection method more easily than a polymerase chain reaction that requires a temperature cycle including temperature rise and fall.

### 5. Example

### (5-1) Production of DNA Aptamer

A DNA aptamer having a DNA sequence of SEQ ID NO: 1 was produced. The DNA aptamer corresponds to the nucleic acid aptamer 1A-1 and also to the binding substance 1. The DNA aptamer of this example was a partially modified version of the DNA aptamer described in Anal. Chem. 2020, 92, 9895-9900.

The DNA sequence of SEQ ID NO: 1 includes a DNA sequence of SEQ ID NO: 2. Anal. Chem. 2020, 92, 9895-9900 states that the DNA sequence of SEQ ID NO: 2 is identified as a DNA aptamer in which an RBD region in a spike glycoprotein of the novel coronavirus SARS-CoV-2 (hereafter referred to as the RBD region) is the target substance 33.

The DNA sequence of SEQ ID NO: 1 includes a DNA sequence of SEQ ID NO: 3. The DNA sequence of SEQ ID NO: 3 corresponds to a 3' terminal region 9. The 3' terminal region 9 is a region assigned to complement the single-stranded cyclic DNA 21A-1.

The DNA aptamer of this example was dissolved in a phosphate buffer solution to produce a DNA aptamer solution. The final concentration of the DNA aptamer in the DNA aptamer solution was 10 µM. The phosphate buffer solution contained 0.05% (v/v) of a surfactant Tween 20. The phosphate buffer solution contained 137 mM of NaCl, 8.1 mM of Na₂HPO₄, 2.7 mM of KCl, and 1.47 mM of KH₂PO₄. The above phosphate buffer solution will be referred to as 1x PBS/T in the following.

### (5-2) Production of Single-Stranded Cyclic DNA

The single-stranded DNA was prepared. The 5' end of the single-stranded DNA was phosphorylation-modified. The phosphorylation-modified single-stranded DNA had a DNA sequence of SEQ ID NO: 4.

The phosphorylation-modified single-stranded DNA was cyclized by the action of CircLigase II to produce the single-stranded cyclic DNA 21A-1.

Next, the single-stranded DNA remaining without cyclization was decomposed by treatment with Exonuclease I, and the single-stranded cyclic DNA 21A-1 was purified and extracted. Note that the single-stranded cyclic DNA 21A-1 included the DNA sequence of SEQ ID NO: 5. The DNA sequence of SEQ ID NO: 5 is the same sequence as the second primer sequence used in the rolling circle amplification.

### (5-3) First Verification

It was verified whether rolling circle amplification proceeded using a DNA aptamer. First, a mixed solution X was produced. The mixed solution X contained phi29 DNA polymerase (NEB Inc., M0269) and SYBR Green I (Takara Bio Inc., 5761A). In more detail, the mixed solution X contained a 10x phi29 buffer (buffer) (final concentration of 1x), a dNTP mix (final concentration of 0.2 mM), BSA (final concentration of 0.1 mg/mL), SYBR Green I (TaKaRa Bio Inc., 5761A) (final concentration of 1x), ROX dye (final concentration of 1x), phi29 DNA polymerase (final concentration of 0.05 U/µL), the single-stranded cyclic DNA produced in (3-2) above (final concentration of 100 nM), a second primer (final concentration of 1 µM), the DNA aptamer produced in (3-1) above (final concentration of 10 nM), and pure water. The volume of the mixed solution X was 20 µL.

Next, the mixed solution X was produced. The mixed solution Y basically had the same composition as the mixed solution X, but did not contain the DNA aptamer and instead contained pure water.

Next, the respective mixed solutions X and Y were incubated for 3 hours at 37°C using a StepOnePlus real-time PCR system (Thermo Fisher Scientific Inc.). During the incubation, the fluorescence kinetics of SYBR Green I was measured in real time.

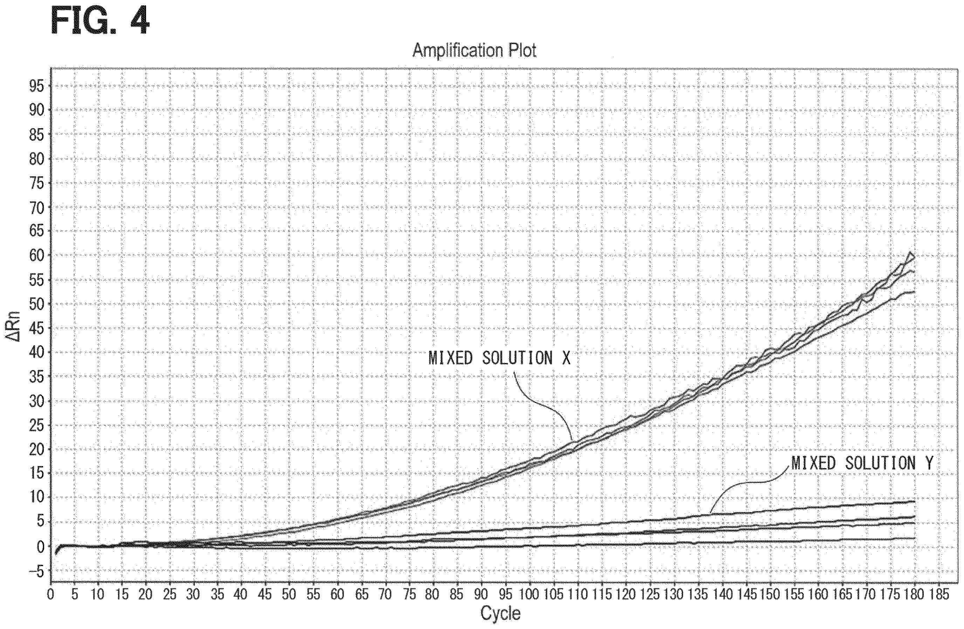

The measurement results are shown in FIG. 4. In the mixed solution X, an increase in fluorescence intensity over time was observed. On the other hand, no increase in fluorescence intensity was observed in the mixed solution Y.

The results of the above measurement indicate the following. The DNA aptamer produced in (5-1) above had the activity to form the complex 63 by hybridizing through the formation of the base pair 61 together with the single-stranded cyclic DNA 21A-1. Using the formed complex 63 as the starting point, the rolling circle amplification occurred, resulting in DNA amplification.

### (5-4) Second Verification

It was verified whether the rolling circle amplification decreases a pH of the mixed solution Z described below. A mixed solution X was produced. The composition of the mixed solution Z was basically the same as that of the mixed solution X, but instead of 10x phi29 buffer, it contained 2x master mix. The 2x master mix contained Tris (final concentration of 2 mM), MgCl₂ (final concentration of 20 mM), (NH₄)₂SO₄ (final concentration of 20 mM), and DTT (final concentration of 8 mM).

There were six types of 2x master mixes. The six 2x master mixes differed only in pH. The pHs of the six 2x master mixes were 7.0, 7.5, 8.0, 8.5, 9.0, and 10.0. The pH was measured by titration with NaOH. Six types of mixed solutions Z were also provided, depending on the type of 2x master mix to be blended.

Next, each of the six types of mixed solutions Z was incubated at 37°C for 3 hours using the StepOnePlus real-time PCR system (Thermo Fisher Scientific Inc.). During the incubation, the fluorescence kinetics of SYBR Green I was measured in real time.

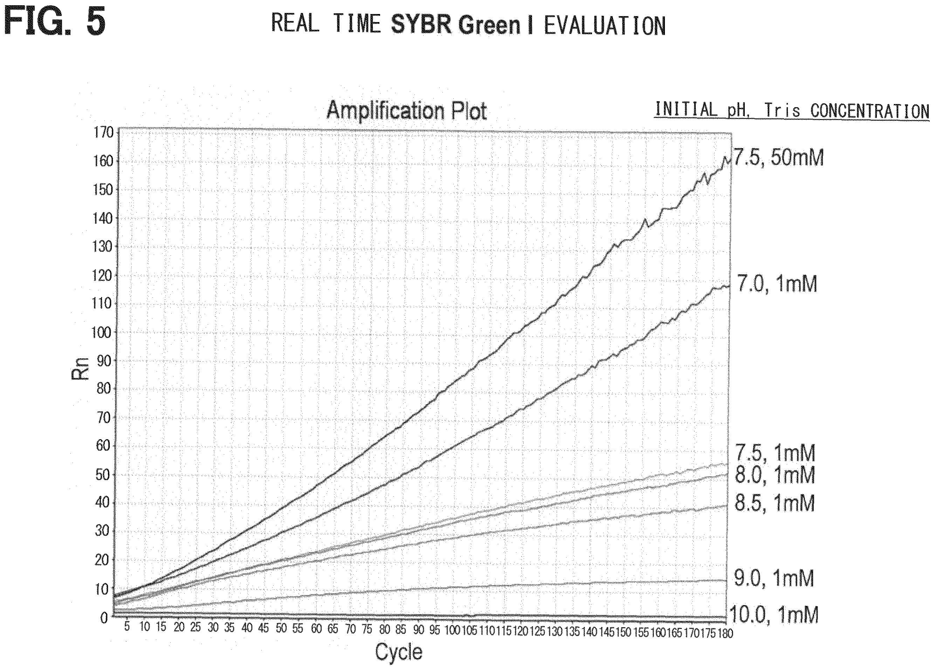

The measurement results are shown in FIG 5. The increase in fluorescence intensity over time was highest when the pH of the 2x master mix blended in the mixed solution Z was 7.0, and it decreased in order of pH from 7.5, 8.0, 8.5, 9.0, to 10.0.

In addition, the pH of the mixed solution Z was measured after the incubation at 37°C for 3 hours. A compact pH meter LAQUAtwin (HORIBA, Ltd., product number pH-33B) was used to measure the pH. The amount of decrease in pH was calculated by subtracting the pH of the mixed solution Z after the incubation from the pH of the mixed solution Z before the incubation.

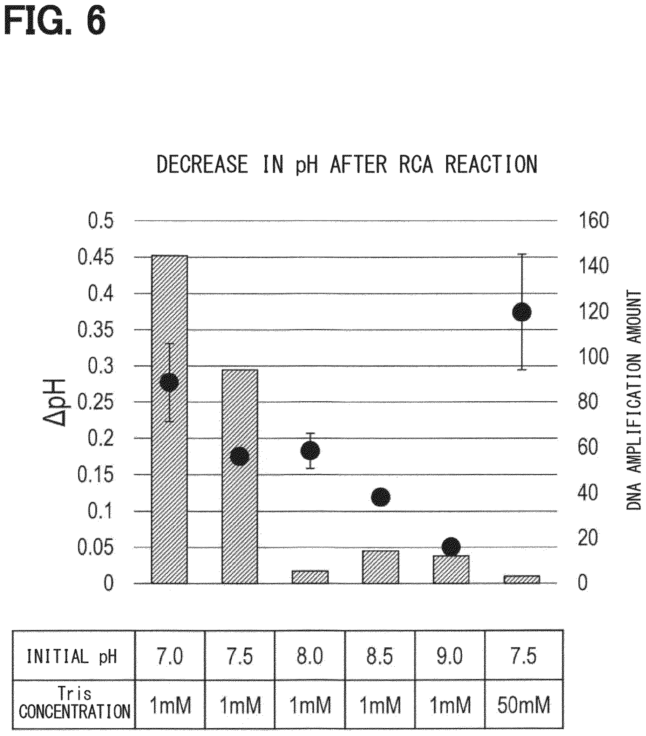

The amount of decrease in pH is shown in FIG. 6. The amount of decrease in pH was highest when the pH of the 2x master mix blended in the mixture Z was 7.0, and it decreased in order of pH of 7.5, 8.5, 9.0, and 8.0.

The results of the above measurement indicate the following. The DNA aptamer had the activity to form the complex 63 by hybridizing through the formation of the base pair 61 together with the single-stranded cyclic DNA 21A-1. Using the formed complex 63 as the starting point, the rolling circle amplification occurred, resulting in DNA amplification. With the incorporation of nucleotides that occurs in the DNA amplification, hydrogen ions were generated, lowering the pH of the mixed solution Z.

### (5-5) Implementation of Analysis Method

Spike S1-His Recombinant Protein (Sino Biological, Inc., product number 40591-V08H) (hereafter referred to as S1) was used as the target substance 33. S1 is a protein containing the RBD region in its amino acid sequence. The DNA aptamer produced in (5-1) above has binding activity to S1.

S1 was dissolved in 0.1 M of carbonate buffer solution to produce an S1 solution. The pH of the 0.1 M of carbonate buffer solution was adjusted to 9.6. The final concentrations of S1 in the S1 solution took two values, namely, 5 µg/mL and 1 µg/mL.

In addition, α-amylase (Lee Biosolutions, Inc., product number 120-17) was dissolved in 0.1 M of carbonate buffer solution to produce an α-amylase solution. The final concentration of α-amylase in the α-amylase solution was 5 µg/mL. The DNA aptamer produced in (5-1) above has no binding activity to α-amylase.

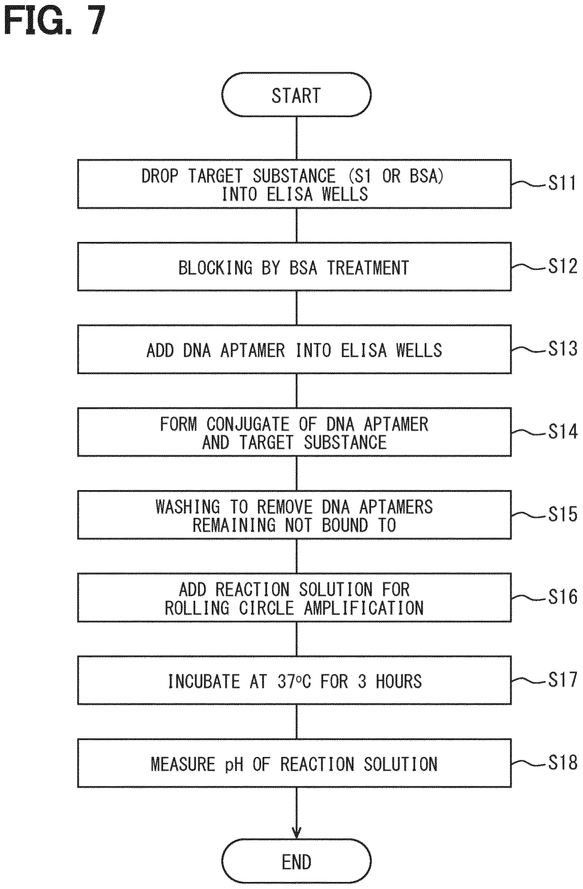

The analysis method was then implemented using the procedure shown in FIG. 7. In the analysis method, it was verified whether the pH of the reaction solution decreased or not due to rolling circle amplification driven after the binding of the DNA aptamer and the target substance.

First, in S11, 100 µL of a solution containing the target substance was dropped into ELISA wells of a 96-well plate H type for ELISA (Sumitomo Bakelite Co., Ltd., product number MS-8896F) and allowed to stand at 4°C overnight. The solution containing the target substance was an S1 solution or α-amylase solution. The target substance was S1 or α-amylase. At this time, the target substance adhered to the ELISA wells. Next, the ELISA wells to which the target substance adhered were washed with 200 µL of 1x PBS/T. The washing was performed three times.

Next, in S12, bovine serum-derived albumin (FUJIFILM Wako Pure Chemical Corporation, product number 013-15104) was dissolved in 1x PBS/T to produce a BSA solution. Bovine serum-derived albumin is hereafter referred to as BSA. The concentration of BSA in the BSA solution was 3% (w/v). Then, 200 µL of BSA solution was dropped into the ELISA wells into which the target substance adhered, and the wells were allowed to stand for 2 hours at room temperature. At this time, blocking was performed. The ELISA wells in which blocking was performed were washed with 200 µL of 1x PBS/T. The washing was performed three times.

Next, in S13, 50 µL of the DNA aptamer solution produced in (5-1) above was dropped into the blocked ELISA wells and allowed to stand for 1 hour at room temperature. At this time, in S14, some of the DNA aptamers were bound to S1 to form the conjugates 65.

Next, in S15, the ELISA wells were washed with 200 µL of 1x PBS/T. The washing was performed three times. At this time, the DNA aptamers that were not bound to the target substance were removed.

Next, in S16, one of the mixed solutions Z in which the blended 2x master mix had a pH of 7.0 was dropped into the ELISA wells for rolling circle amplification. Then, in S17, the dropped mixture Z was incubated at 37°C for 3 hours.

Next, in S18, the pH of the mixed solution Z after the incubation was measured. A compact pH meter LAQUAtwin (HORIBA, Ltd., product number pH-33B) was used to measure the pH. The measurement results in pH is shown in Table 1.

**[Table 1]**

| Solution containing target substance | Amount of change in pH | Standard deviation (±SD) |
|---|---|---|
| S1 5µg/mL | -0.48 | 0.03 |
| S1 1µg/mL | -0.27 | 0.05 |
| α-amylase 5µg/mL | -0.03 | 0.03 |
| None | 0.00 | 0.04 |

When the target substance was S1, the pH of the reaction solution decreased due to rolling circle amplification driven after the binding of the DNA aptamer and the target substance. Therefore, S1 was able to be detected by detecting the phenomenon of the decrease in pH. On the other hand, when the target substance was α-amylase, the pH of the reaction solution did not decrease.

### 6. Other Embodiments

The embodiments of the present disclosure have been described above, but the present disclosure is not limited to the embodiments described above and can be implemented with various variations.

(1) A plurality of functions associated with one component in the above embodiments may be implemented by a plurality of components, or one function associated with one component may be implemented by a plurality of components. A plurality of functions associated with a plurality of components may be implemented by a single component, or a single function implemented by a plurality of components may be implemented by a single component. Some of the configurations of the above embodiments may be omitted. Also, at least a portion of the configuration of the above embodiments may be added or substituted for other configurations of the above embodiments.
(2) In addition to the analysis method described above, the present disclosure can also be implemented in various forms, such as binding substances and producing methods for binding substances.

### [Technical ideas disclosed by the present specification]

### [Item 1]

An analysis method comprising: mixing a binding substance (1, 1A, 1A-1, 1B) and a sample (31) including a target substance (33), the binding substance including a nucleic acid region (1A, 1A-1, 3) composed of a nucleic acid and having activity to bind to the target substance;
removing the binding substance not bound to the target substance;
amplifying the nucleic acid region; and
detecting a phenomenon caused by the amplification of the nucleic acid region.

### [Item 2]

The analysis method according to Item 1, wherein
after the binding substance not bound to the target substance is removed, the binding substance and a template nucleic acid (21, 21A, 21A-1) including a sequence complementary to a 3' side sequence of the nucleic acid region form a complex, and the nucleic acid region is amplified by an action of a nucleic acid amplifying enzyme using the complex (63) as a starting point.

### [Item 3]

The analysis method according to Item 2, wherein
the nucleic acid region is amplified by a rolling circle amplification method, and
the template nucleic acid is a single-stranded cyclic nucleic acid (21A).

### [Item 4]

The analysis method according to any one of Items 1 to 3, wherein
the phenomenon is at least one of generation of hydrogen ions associated with incorporation of a nucleotide by the amplification of the nucleic acid region and a pyrophosphoric acid associated with incorporation of the nucleotide by the amplification of the nucleic acid region.

### [Item 5]

The analysis method according to any one of Items 1 to 4, wherein
when the phenomenon is the generation of pyrophosphoric acid, a change in oxidation-reduction potential caused by a reaction cascade driven by the generated pyrophosphoric acid is detected using a potentiometer (51), and
when the phenomenon is production, consumption, or absorption of hydrogen ions, the phenomenon is detected using a pH meter (51).

### [Item 6]

The analysis method according to any one of Items 1 to 5, wherein
the nucleic acid region is amplified by an isothermal nucleic acid amplification method in which a reaction proceeds at a constant temperature without requiring a temperature cycle.

### [Item 7]

The analysis method according to any one of Items 1 to 6, wherein
the nucleic acid region is amplified by a rolling circle amplification method,
a reaction solution for amplifying the nucleic acid region contains a Tris buffer solution at a final concentration of 0 mM or more and 10 mM or less,
a pH of the reaction solution is 7.0 or higher and 9.0 or lower, and
the phenomenon is detected using a pH meter (51).

### [Item 8]

The analysis method according to any one of Items 1 to 7, wherein
the target substance is a protein, sugar, lipid, nucleic acid, or low molecular weight compound.

## Claims

1. An analysis method comprising:
mixing a binding substance (1, 1A, 1A-1, 1B) and a sample (31) including a target substance (33), the binding substance including a nucleic acid region (1A, 1A-1, 3) composed of a nucleic acid and having activity to bind to the target substance;
removing the binding substance not bound to the target substance;
amplifying the nucleic acid region; and
detecting a phenomenon caused by the amplification of the nucleic acid region.

2. The analysis method according to claim 1, wherein
after the binding substance not bound to the target substance is removed, the binding substance and a template nucleic acid (21, 21A, 21A-1) including a sequence complementary to a 3' side sequence of the nucleic acid region form a complex, and
the nucleic acid region is amplified by an action of a nucleic acid amplifying enzyme using the complex (63) as a starting point.

3. The analysis method according to claim 2, wherein
the nucleic acid region is amplified by a rolling circle amplification method, and
the template nucleic acid is a single-stranded cyclic nucleic acid (21A).

4. The analysis method according to any one of claims 1 to 3, wherein
the phenomenon is at least one of generation of hydrogen ions associated with incorporation of a nucleotide by the amplification of the nucleic acid region and a pyrophosphoric acid associated with incorporation of the nucleotide by the amplification of the nucleic acid region.

5. The analysis method according to claim 1 or 2, wherein
when the phenomenon is the generation of pyrophosphoric acid, a change in oxidation-reduction potential caused by a reaction cascade driven by the generated pyrophosphoric acid is detected using a potentiometer (51), and
when the phenomenon is production, consumption, or absorption of hydrogen ions, the phenomenon is detected using a pH meter (51).

6. The analysis method according to claim 1 or 2, wherein
the nucleic acid region is amplified by an isothermal nucleic acid amplification method in which a reaction proceeds at a constant temperature without requiring a temperature cycle.

7. The analysis method according to claim 1 or 2, wherein
the nucleic acid region is amplified by a rolling circle amplification method,
a reaction solution for amplifying the nucleic acid region contains a Tris buffer solution at a final concentration of 0 mM or more and 10 mM or less,
a pH of the reaction solution is 7.0 or higher and 9.0 or lower, and
the phenomenon is detected using a pH meter (51).

8. The analysis method according to claim 1 or 2, wherein
the target substance is a protein, sugar, lipid, nucleic acid, or low molecular weight compound.
